# EUROPEAN PATENT APPLICATION

(11) **EP 4 030 343 A1**
(43) Date of publication of application: **20.07.2022**
(21) Application number: 20879311.7
(22) Date of filing: 13.10.2020
(51) Int. Cl.: G06K 9/62

(54) **FACIAL SKIN DETECTION METHOD AND APPARATUS**

(30) Priority: 22.10.2019 CN 201911005689
(71) Applicant: Huawei Technologies Co., Ltd., Longgang District Shenzhen, Guangdong 518129 (CN)
(72) Inventor: ZHAO, Lin, Shenzhen, Guangdong 518129 (CN); DING, Xin, Shenzhen, Guangdong 518129 (CN); DONG, Chen, Shenzhen, Guangdong 518129 (CN); GAO, Wenmei, Shenzhen, Guangdong 518129 (CN); LU, Yuewan, Shenzhen, Guangdong 518129 (CN)
(74) Representative: Pfenning, Meinig & Partner mbB
(86) International application number: PCT/CN2020/120551
(87) International publication number: WO 2021/078037

(57) **Abstract**

This application provides a facial skin detection method and apparatus. The method is performed by a terminal device having a front-facing camera. After a first image including a face is obtained, feature information of pores in a target area of the face is extracted, and the pores in the target area are further classified into a plurality of pore categories based on the feature information of the pores in the target area. Finally, feature information of pores of each pore category is input into at least one pore detection model, to obtain pore detection data of each pore category. Therefore, a skin detection result of the face can be determined based on pore detection data of the plurality of pore categories. This application resolves a technical problem in the conventional technology that the terminal device cannot perform skin detection based on a face image photographed by the front-facing camera.

## Description

This application claims priority to Chinese Patent Application No. 201911005689.6, filed with the China National Intellectual Property Administration on October 22, 2019 and entitled "FACIAL SKIN DETECTION METHOD AND APPARATUS", which is incorporated herein by reference in its entirety.

### TECHNICAL FIELD

This application relates to the field of terminal technologies, and in particular, to a facial skin detection method and apparatus.

### BACKGROUND

With the progress of science and technology and the development of medical technology, cosmetology science has entered a stage of rapid development. Dermatology clinics in hospitals and medical cosmetology institutions related to cosmetology have been popularized to provide comprehensive diagnosis and treatment and follow-up cosmetology services for users with skin-related needs. For example, the user may go to a hospital, and a medical staff may perform comprehensive evaluation and diagnosis on the facial skin, and give an evaluation on the facial skin of the user. If the facial skin of the user is not well, a daily routine and an eating habit may be adjusted subsequently based on the evaluation on the skin, or skin treatment may be performed directly in the hospital, so as to implement skin management.

In the conventional technology, to meet a user requirement, an application in some terminal devices such as a mobile phone or a tablet computer provides a skin detection function. After the user takes a photograph of a face by using a camera of the terminal device, the terminal device evaluates the facial skin of the user according to an image processing algorithm. In this way, the user can know skin conditions in real time without going to the hospital.

According to the conventional technology, because a rear-facing camera of the terminal device has a relatively high pixel, and devices such as a flashlight and a focus motor are provided, the face image photographed by the user by using the rear-facing camera of the terminal device has relatively high quality. Various features of the facial skin such as wrinkles, spots and acne can be recognized directly through image processing. However, if the face is photographed by using a front-facing camera of the terminal device, an obtained face image has relatively low quality, and features of the facial skin may not be directly recognized. As a result, the terminal device cannot perform facial skin detection by using the image photographed by the front-facing camera. Therefore, how to enable the terminal device to perform facial skin detection based on the face image photographed by the front-facing camera is a technical problem to be urgently resolved in this field.

### SUMMARY

This application provides a facial skin detection method and apparatus, to overcome a technical problem in the conventional technology that a terminal device cannot perform skin detection on a face based on a face image photographed by a front-facing camera.

According to a first aspect of this application, a facial skin detection method is provided. The method may be performed by a terminal device having a front-facing camera. For the method, after obtaining a first image including a face, the terminal device extracts feature information of pores in a target area of the face, and classifies the pores in the target area into a plurality of pore categories based on the feature information of the pores in the target area. Finally, the terminal device inputs feature information of pores of each pore category into at least one pore detection model, to obtain pore detection data of each pore category. Therefore, the terminal device can determine the skin detection result of the face based on pore detection data of the plurality of pore categories.

In conclusion, according to the facial skin detection method provided in the first aspect of this application, classification detection is performed on the pores based on a feature of the pores that are in the face image photographed by the front-facing camera and that are relatively easy to be recognized, and a skin detection result of the face is finally determined by using the detection data of the pores, so as to resolve a technical problem in the conventional technology that the terminal device cannot perform skin detection based on the face image photographed by the front-facing camera. In addition, this application can enable the terminal device to provide that in different scenarios including the front-facing camera, a user can detect facial skin, and a detection result has certain accuracy and stability. In addition, because the pore detection model and a skin detection model are used, and the method does not depend on a recognition result of a single image, an unreasonable jump does not occur in the detection result when the terminal device performs skin detection on a same face in different scenarios at a same time. In this way, a stable and accurate skin detection result can be provided for the user of the terminal device by using the front-facing camera.

In an embodiment of the first aspect of this application, the feature information of the pores includes one or more of the following: an area, a depth, and a color of the pores.

According to the facial skin detection method provided in the foregoing embodiments of this application, the pores can be classified by using different pieces of feature information of the pores of the face, so as to subsequently detect the pores of different types by using the pore detection model. In this way, facial skin detection can still be performed by using the pores in the low-quality image photographed by using the front-facing camera. Therefore, the facial skin detection method provided in this application can be applied to a scenario photographed by the front-facing camera.

In an embodiment of the first aspect of this application, the classifying the pores in the target area into a plurality of pore categories based on the feature information of the pores in the target area includes: inputting the feature information of all pores into a pore classification model, to obtain scoring data of feature information of each pore by the pore classification model, where the pore classification model is obtained by training the feature information of the pores of the plurality of pore categories; and classifying the pores in the target area into the plurality of categories based on the scoring data of the feature information of each pore.

In the facial skin detection method provided in the foregoing embodiments of this application, after the feature information of all pores in the target area is extracted, the extracted feature information of all pores in the target area of the face may be input into the pore classification model, to obtain the scoring data of feature information of each pore by the pore classification model. Finally, based on the scoring data of the feature information of each pore, all pores in the target area are classified into the plurality of categories. Therefore, the pores can be classified quickly and accurately by using the pore classification model implemented based on machine learning.

In an embodiment of the first aspect of this application, the inputting feature information of pores of each pore category into at least one pore detection model, to obtain detection data of each pore category includes: inputting the feature information of the pores of each pore category into a plurality of pore detection models that are in a one-to-one correspondence with the pore categories, to obtain the detection data of each pore category, where the plurality of pore detection models are obtained by training the feature information of the pores of the corresponding pore categories.

According to the facial skin detection method provided in the foregoing embodiments of this application, the terminal device may store a plurality of pore detection models that are in a one-to-one correspondence with the pore categories, and the terminal device inputs the pores of different categories into the pore detection models of the corresponding pore categories, to obtain detection data corresponding to the pore categories. Because different pore detection models are used to detect the pores of different categories, each pore detection model only needs to detect pores of one category. In this way, a calculation amount of each pore detection model is reduced, and a calculation speed of each pore detection model is improved. When all pore detection models are calculated at the same time, an overall computing speed and computing efficiency can be improved.

In an embodiment of the first aspect of this application, a created image is used as a training image, and regression models of the pores of different categories are obtained by training by using a machine learning method as corresponding pore detection models. A quantitative score of the pores of the category is obtained by using the pore detection model and is used as the pore detection data. The method includes: obtaining a plurality of images including a face, where pores on a face in each image may be classified into the plurality of pore categories; and training, based on feature information of pores of each pore category in the plurality of images, a pore detection model that is in a one-to-one correspondence with the pore category, to obtain a plurality of pore detection models that are in a one-to-one correspondence with the plurality of pore categories.

In an embodiment of the first aspect of this application, the inputting feature information of pores of each pore category into at least one pore detection model, to obtain detection data of each pore category includes: inputting the feature information of the pores of each pore category into a first pore detection model, to obtain the detection data of each pore category, where the first pore detection model is obtained by training the feature information of the pores of all categories.

According to the facial skin detection method provided in the foregoing embodiments of this application, the terminal device may input the obtained feature information of the pores of all different categories into the first pore detection model, to obtain detection data of the pores of each pore category. Therefore, different pores can be classified more intuitively by using one model, and a training amount of the model can be reduced.

In an embodiment of the first aspect of this application, the determining a skin detection result of the face based on pore detection data of the plurality of pore categories includes: inputting the pore detection data of the plurality of pore categories into a skin detection model obtained by training in advance, to obtain the skin detection result of the face, where the skin detection model is established based on a weight coefficient occupied by the at least one pore category in the skin detection result.

In an embodiment of the first aspect of this application, the weight coefficient occupied by the at least one pore category in the skin detection result is obtained through regression model training.

In an embodiment of the first aspect of this application, the skin detection result includes skin delicateness of the face.

In an embodiment of the first aspect of this application, before the extracting feature information of pores in a target area of the face, the method further includes: determining the target area of the face in the first image; sequentially performing filtering processing, enhancement processing, and morphology processing on the target area; and determining the feature information of all pores from the processed target area.

According to the facial skin detection method provided in the foregoing embodiments of this application, after preprocessing the first image, the terminal device extracts the feature information of all pores from the target area of the first image obtained after processing. Accuracy of determining, by the terminal device, the pores in the target area and the extracted feature information of the pores can be improved, and therefore, accuracy of subsequent detection on the facial skin can be ensured. In addition, after the preprocessing process is performed, the terminal device may perform skin detection on the face only by using the target area, and does not need to detect the entire first image. In this way, a calculation amount when the terminal device performs skin detection on the face is reduced, a processing time is reduced, and efficiency of performing facial skin detection by the terminal device can be improved.

In an embodiment of the first aspect of this application, the obtaining a first image including a face includes: obtaining, through photographing by using a front-facing camera, the first image including the face.

In an embodiment of the first aspect of this application, after the determining a skin detection result of the face, the method further includes: determining a problem existing in facial skin and at least one piece of suggestion information for resolving the problem; and displaying, on a display interface, the at least one piece of suggestion information for resolving the problem.

The facial skin detection method provided in the foregoing embodiments of this application can be used for a display interface of the terminal device. When a user shoots a face image by using the front-facing camera, the terminal device may directly display the skin detection result of the face on the display interface after determining the skin detection result of the face based on the face image. In addition, the terminal device may further obtain the suggestion information based on the skin detection result. Therefore, the terminal device can provide a facial skin detection function and a related consultation function for the user. In this way, more functions can be provided by the terminal device during facial skin detection, and user experience of the terminal device is further improved.

In an embodiment of the first aspect of this application, after the determining a skin detection result of the face, the method further includes: sending the skin detection result to a server, where the server is configured for medical personnel to diagnose the problem existing in the facial skin; receiving the at least one piece of suggestion information for resolving the problem that is sent by the server; and displaying, on a display interface, the at least one piece of suggestion information for resolving the problem.

The facial skin detection method provided in the foregoing embodiments of this application can be used for the display interface of the terminal device. When the user shoots a face image by using the front-facing camera, the terminal device may directly display the skin detection result of the face on the display interface after determining the skin detection result of the face based on the face image. In addition, the terminal device may further send the detection result to the server, so that the medical personnel can inquire about a problem existing in the facial skin, and propose suggestion information based on the skin detection result, and the terminal device can finally display the suggestion information sent by the server. Therefore, a facial skin detection function and a related consulting function are provided for the user. In this way, more functions can be provided by the terminal device during facial skin detection, and user experience of the terminal device can also be improved.

In an embodiment of the first aspect of this application, the first image includes a plurality of faces. The skin detection result of the face includes: a skin detection result that is in a one-to-one correspondence with each face in the first image.

In an embodiment of the first aspect of this application, the method further includes: displaying the first image on the display interface, and displaying, at a position corresponding to each face in the first image, the skin detection result that is in a one-to-one correspondence with each face.

In the facial skin detection method provided in the foregoing embodiments of this application, after obtaining the image including the plurality of faces, the terminal device may detect the skin of each face in the image, and display the skin detection result of each face on the display interface at the same time after obtaining the skin detection result of each face. In this way, after photographing faces with a family member and a friend by using the front-facing camera of the terminal device, the user can directly view a skin detection result of each person by using the display interface of the terminal device. In this way, more functions can be provided by the terminal device during facial skin detection, interest is added when the user uses the facial skin detection, and user experience of the terminal device can also be improved.

According to a second aspect of this application, a facial skin detection apparatus is provided. The facial skin detection apparatus is configured to perform the facial skin detection method provided in the first aspect of this application. The apparatus may be an electronic device. The facial skin detection apparatus includes an obtaining module, an extraction module, a classification module, a pore detection module, and a skin detection module. The obtaining module is configured to obtain a first image including a face. The extraction module is configured to extract feature information of pores in a target area of the face. The classification module is configured to classify the pores in the target area into a plurality of pore categories based on the feature information of the pores in the target area. The pore detection module is configured to input feature information of pores of each pore category into at least one pore detection model to obtain pore detection data of each pore category. The skin detection module 1705 is configured to determine a skin detection result of the face based on pore detection data of the plurality of pore categories.

In an embodiment of the second aspect of this application, the feature information of the pores includes one or more of the following: an area, a depth, and a color of the pores.

In an embodiment of the second aspect of this application, the classification module is specifically configured to: input the feature information of all pores into a pore classification model, to obtain scoring data of the feature information of each pore by the pore classification model, where the pore classification model is obtained by training the feature information of the pores of the plurality of pore categories; and the pores in the target area are classified into a plurality of categories based on the scoring data of the feature information of each pore.

In an embodiment of the second aspect of this application, the pore detection module is specifically configured to input the feature information of the pores of each pore category into the plurality of pore detection models that are in a one-to-one correspondence with the pore categories, to obtain detection data of each pore category. The plurality of pore detection models are obtained by training the feature information of the pores of the corresponding pore categories.

In an embodiment of the second aspect of this application, the facial skin detection apparatus further includes a training module. The obtaining module is further configured to obtain a plurality of images including a face, where pores on a face in each image may be classified into the plurality of pore categories. The training module is configured to train, based on feature information of pores of each pore category in the plurality of images, a pore detection model that is in a one-to-one correspondence with the pore category, to obtain a plurality of pore detection models that are in a one-to-one correspondence with the plurality of pore categories.

In an embodiment of the second aspect of this application, the pore detection module is specifically configured to input the feature information of the pores of each pore category into a first pore detection model, to obtain detection data of each pore category, where the first pore detection model is obtained by training the feature information of the pores of all categories.

In an embodiment of the second aspect of this application, the skin detection module is specifically configured to input the pore detection data of the plurality of pore categories into a skin detection model obtained by training in advance, to obtain the skin detection result of the face, where the skin detection model is established based on a weight coefficient occupied by the at least one pore category in the skin detection result.

In an embodiment of the second aspect of this application, the weight coefficient occupied by the at least one pore category in the skin detection result is obtained through regression model training.

In an embodiment of the second aspect of this application, the skin detection result includes skin delicateness of the face.

In an embodiment of the second aspect of this application, the extraction module is further configured to determine the target area of the face in the first image, sequentially perform filtering processing, enhancement processing and morphology processing on the target area, and determine the feature information of all pores from the processed target area.

In an embodiment of the second aspect of this application, the obtaining module is specifically configured to obtain, by using the front-facing camera, the first image including the face.

In an embodiment of the second aspect of this application, the facial skin detection apparatus further includes a display module. The skin detection module is further configured to determine a problem existing in the skin of the face and at least one piece of suggestion information for resolving the problem; and the display module is further configured to display, on a display interface, the at least one piece of suggestion information for resolving the problem.

In an embodiment of the second aspect of this application, the facial skin detection apparatus further includes a display module and a transceiver module. The transceiver module is configured to send the skin detection result to a server, where the server is configured for medical personnel to diagnose the problem existing in the facial skin. The transceiver module is further configured to receive the at least one piece of suggestion information for resolving the problem that is sent by the server. The display module is configured to display, on a display interface, the at least one piece of suggestion information for resolving the problem.

In an embodiment of the second aspect of this application, the first image includes a plurality of faces. The skin detection result of the face includes: a skin detection result that is in a one-to-one correspondence with each face in the first image.

In an embodiment of the second aspect of this application, the display module is further configured to: display the first image on the display interface, and display, at a position corresponding to each face in the first image, the skin detection result that is in a one-to-one correspondence with each face.

For beneficial effects of the second aspect of this application, refer to beneficial effects of the embodiments of the first aspect. Details are not described herein again.

According to a third aspect, this application provides a facial skin detection apparatus. The facial skin detection apparatus may be the terminal device in the foregoing method embodiments, and performs the facial skin detection method in the first aspect of this application. The facial skin detection apparatus includes a memory, a communications interface, and a processor. The memory is configured to store a computer program or instructions. The processor is coupled to the memory and the communications interface. When the processor executes the computer program or the instructions, the communications apparatus is enabled to perform the facial skin detection method according to the first aspect.

According to a fourth aspect, this application provides a computer program product. The computer program product includes computer program code, and when the computer program code is run on a computer, the computer is enabled to perform the facial skin detection method according to the first aspect.

According to a fifth aspect, this application provides a chip system. The chip system includes a processor, configured to perform the facial skin detection method in the first aspect, for example, receive or process data and/or information in the foregoing method. In a possible design, the chip system further includes a memory. The memory is configured to store program instructions and/or data. The chip system may include a chip, or may include a chip and another discrete device.

According to a sixth aspect, this application provides a computer-readable storage medium. The computer-readable storage medium stores computer programs, where when the computer programs are run, the facial skin detection method according to the first aspect is implemented.

### BRIEF DESCRIPTION OF DRAWINGS

FIG. 1 is a schematic diagram of a terminal device to which this application is applied;
FIG. 2 is a schematic diagram of an application in which a terminal device performs facial skin detection in the conventional technology;
FIG. 3 shows a market survey of a facial skin detection application in the conventional technology;
FIG. 4 is a schematic flowchart of an embodiment of a facial skin detection method according to this application;
FIG. 5 is a schematic diagram of a manner of obtaining a first image including a face by a terminal device according to this application;
FIG. 6 is a schematic diagram of a first image including a face according to this application;
FIG. 7 is a schematic diagram of extracting a target area in a first image according to this application;
FIG. 8 is a schematic diagram of a target area extracted from a first image according to this application;
FIG. 9 is a schematic diagram of pores in a target area of a first image according to this application;
FIG. 10 is a schematic diagram of pores of a plurality of categories according to this application;
FIG. 11 is a schematic diagram of pores that are classified by a pore classification model according to this application;
FIG. 12 is a schematic diagram of a structure of a pore detection model according to this application;
FIG. 13 is a schematic flowchart of image preprocessing in a facial skin detection method according to this application;
FIG. 14A, FIG. 14B, FIG. 14C, and FIG. 14D are schematic diagrams of image preprocessing according to this application;
FIG. 15A, FIG. 15B1, and FIG. 15B2 are schematic diagrams of an embodiment of a method for displaying a display interface of a terminal device according to this application;
FIG. 16A, FIG. 16B1, and FIG. 16B2 are schematic diagrams of another embodiment of a method for displaying a display interface of a terminal device according to this application;
FIG. 17 is a schematic diagram of a structure of an embodiment of a facial skin detection apparatus according to this application;
FIG. 18 is a schematic diagram of a structure of an embodiment of a facial skin detection apparatus according to this application;
FIG. 19 is a schematic diagram of a structure of an embodiment of a facial skin detection apparatus according to this application; and
FIG. 20 is a schematic diagram of a structure of an embodiment of a facial skin detection apparatus according to this application.

### DESCRIPTION OF EMBODIMENTS

Before formally describing the embodiments of this application, an application scenario of this application and a problem existing in an existing application are first described with reference to the accompanying drawings.

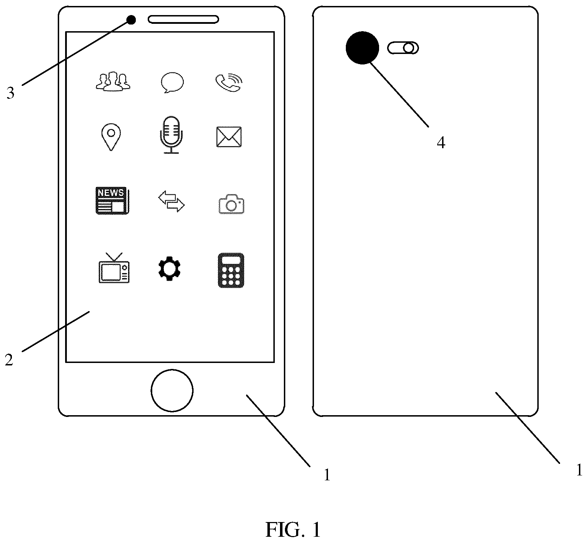

For example, FIG. 1 is a schematic diagram of a terminal device to which this application is applied. The terminal device 1 shown in FIG. 1 may be any electronic device that has a display interface 2, a front-facing camera 3, and a rear-facing camera 4, such as a mobile phone, a tablet computer, or a notebook computer. In FIG. 1, an example in which the terminal device 1 is a mobile phone is used for description, but the terminal device 1 is not limited.

The front-facing camera 3 and the rear-facing camera 4 of the terminal device 1 are disposed on different surfaces. The front-facing camera 3 is a camera that is on the terminal device 1 and that is on a same side as the display interface 2. When a user performs photographing by using the front-facing camera 3 of the terminal device 1, a photographing interface of the front-facing camera 3 may be directly viewed in real time by using the display interface 2. The rear-facing camera 4 is a camera that is on the terminal device 1 and that is not on a same side as the display interface 2. When photographing by using the rear-facing camera 4 of the terminal device 1, the user cannot directly view a photographing interface of the rear-facing camera 4 in real time by using the display interface 2, and needs to flip the terminal device 1 after photographing is completed, to view, from the display interface 2, an image that has been photographed by the rear-facing camera 4.

With improved performance of the terminal device and continuous development of a medical cosmetology technology, some applications installed in the terminal device provide a facial skin detection function, to meet a requirement of the user of the terminal device for facial skin detection and management.

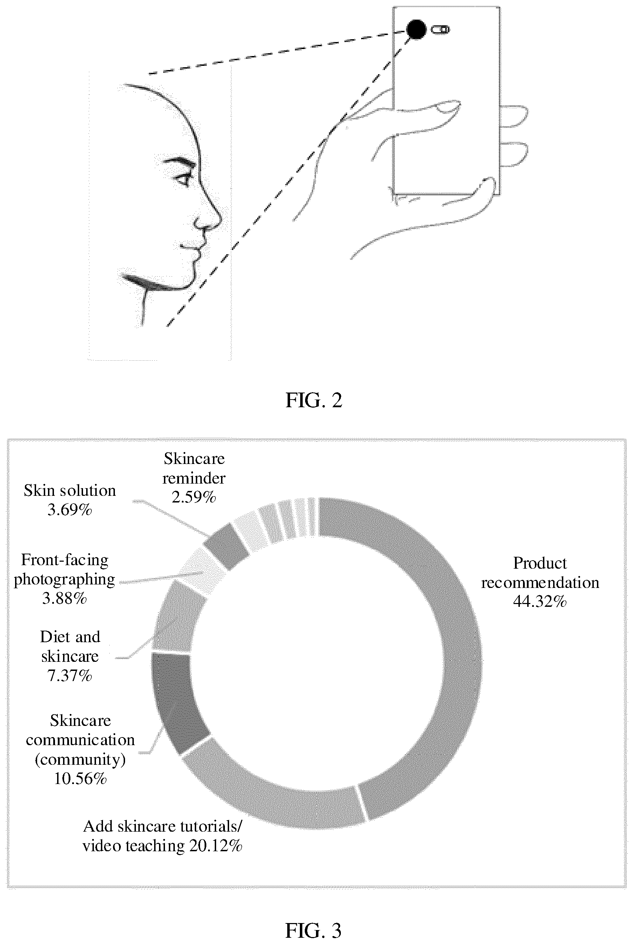

For example, FIG. 2 is a schematic diagram of an application in which a terminal device performs facial skin detection in the conventional technology. As shown in FIG. 2, after a user takes a picture of a face by using a rear-facing camera of the terminal device, the terminal device evaluates facial skin of the user according to an image processing algorithm. In this way, the user can know skin condition in real time without going to the hospital. Particularly, because the rear-facing camera of the terminal device has a relatively high pixel, and devices such as a flashlight and a focus motor are provided, the face image photographed by the user by using the rear-facing camera of the terminal device has relatively high quality. Various features of the facial skin such as wrinkles, spots and acne can be recognized directly through image processing. Therefore, in the conventional technology shown in FIG. 2, when using the terminal device to perform facial skin detection, the user first needs to start an application corresponding to skin detection on a display interface of the terminal device. Then, the user flips the terminal device, so as to aim the rear-facing camera at the face and take a picture of the face to obtain an image including the face. Finally, the terminal device performs detection based on the face image and generates a detection result. In this case, the user further needs to flip the terminal device to view a skin detection result on the display interface.

In other words, in the conventional technology shown in FIG. 2, when the user performs facial skin detection by using the terminal device, because the face image is photographed by using the rear-facing camera, the terminal device needs to be flipped at least twice in an entire process. This brings some inconvenience to use of the user. Therefore, in a market survey conducted on a facial skin detection application shown in FIG. 3, 3.69% of users expect that the facial skin detection application may have a function of photographing by using the front-facing camera of the terminal device. FIG. 3 shows a market survey of a facial skin detection application in the conventional technology. Therefore, the facial skin detection application of the terminal device needs to have a front-facing photographing function.

However, because the front-facing camera of the terminal device usually has a relatively low pixel and does not have a device such as a flash, a face image obtained by photographing has relatively low quality, and excessive skin features in the face image may not be directly recognized. As a result, the terminal device cannot detect the facial skin by using the image photographed by the front-facing camera. Therefore, how to enable the terminal device to perform skin detection on the face based on the face image photographed by the front-facing camera is a technical problem to be urgently resolved in this field.

Therefore, this application provides a facial skin detection method and apparatus. Classification detection is performed on the pores based on a feature of pores that are in the face image photographed by the front-facing camera and that are relatively easy to be recognized, and a skin detection result of the face is finally determined by using detection data of the pores, so as to resolve a technical problem in the conventional technology that the terminal device cannot perform skin detection based on the face image photographed by the front-facing camera.

The following describes the facial skin detection method and apparatus provided in this application with reference to accompanying drawings.

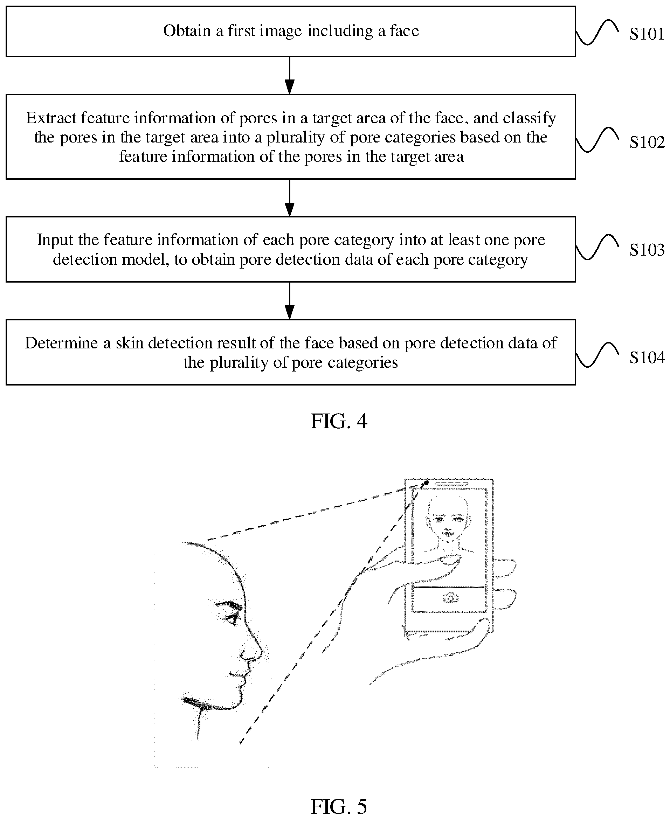

FIG. 4 is a schematic flowchart of an embodiment of a facial skin detection method according to this application. The facial skin detection method provided in this embodiment may be executed by the terminal device shown in FIG. 1 or by a chip in the terminal device. In the embodiments of this application, a terminal device is used as an example for description. The facial skin detection method shown in FIG. 4 includes the following steps.

S101: Obtain a first image including a face.

Specifically, when performing facial skin detection, the terminal device first obtains, by using S101, the first image including the face, and detects facial skin in the first image in an image processing manner.

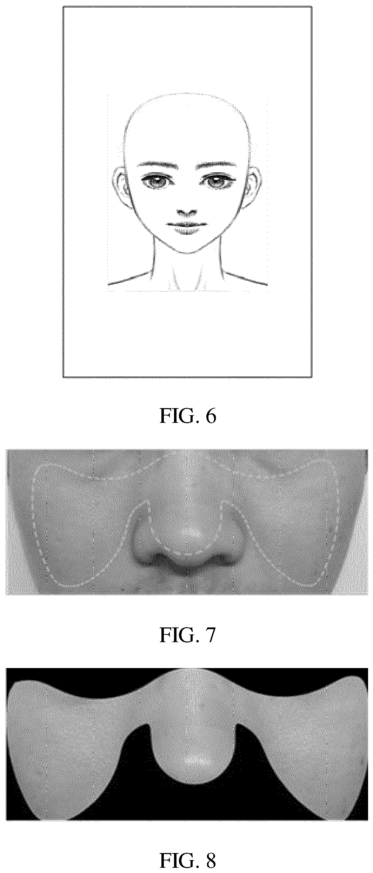

Optionally, in S101, the terminal device may specifically obtain, through photographing by using the front-facing camera, the first image including the face. For example, FIG. 5 is a schematic diagram of a manner of obtaining a first image including a face by the terminal device according to this application. After starting an application corresponding to skin detection on a display interface of the terminal device, the user may directly aim at the face by using the front-facing camera on a same side as the display interface to perform photographing. In this case, after detecting that the user taps a photographing control on the display interface, the terminal device may photograph the face of the user to obtain the first image including the face. For example, FIG. 6 is a schematic diagram of a first image including a face according to this application. The first image includes at least one face.

Alternatively, the terminal device may detect content that can be photographed by the front-facing camera in real time, and when determining that a face appears in a photographing range of the front-facing camera, perform photographing to obtain the first image including the face. Alternatively, the terminal device may further detect whether a face that appears in a photographing range of the camera meets a condition. The condition may be current illumination or a distance between the face and the camera. After the condition is met, the terminal device may perform photographing to obtain the first image including the face.

Alternatively, optionally, the first image obtained by the terminal device in S101 may be an image that is previously photographed and stored by the terminal device. For example, the terminal device invokes, according to an instruction of a user, the first image that includes the face and that is stored in the storage device. Alternatively, the terminal device may receive, by using a communications interface (wireless fidelity (wireless fidelity, WiFi), Bluetooth, or the like), the first image that includes the face and that is sent by another device.

S102: Extract feature information of pores in a target area of the face, and classify the pores in the target area into a plurality of pore categories based on the feature information of the pores in the target area.

Subsequently, in S102, the terminal device further processes the first image shown in FIG. 6 obtained in S101, specifically extracts the feature information of the pores in the target area of the face, and classifies the pores based on the feature information of the pores.

The target area may be an area in which pores are most likely to appear on the face, or may be an area pre-stored in the terminal device, or may be an area specified by the user. The target area may also be referred to as a region of interest (region of interest, ROI). For example, FIG. 7 is a schematic diagram of extracting a target area in a first image according to this application. In the example shown in FIG. 7, a nose on the face and facial areas on two sides of the nose that are prestored in a terminal device are the target area. After obtaining the first image including the face, the terminal device may detect the face, further extract 15 key feature points of the face from the first image, connect the 15 key feature points into a curve, use the inside of the closed curve shown in FIG. 7 as the target area of the face in the first image, and extract only the feature information of the pores in the target area.

FIG. 8 is a schematic diagram of a target area extracted from a first image according to this application. FIG. 8 shows a target area obtained after the first image shown in FIG. 6 is extracted in the manner shown in FIG. 7. In subsequent processing, the terminal device may perform skin detection on the face only based on the target area shown in FIG. 8, and does not need to perform detection on the entire first image. In this way, a calculation amount when the terminal device performs skin detection on the face is reduced, so as to reduce a processing time, and efficiency of performing facial skin detection by the terminal device can be further improved.

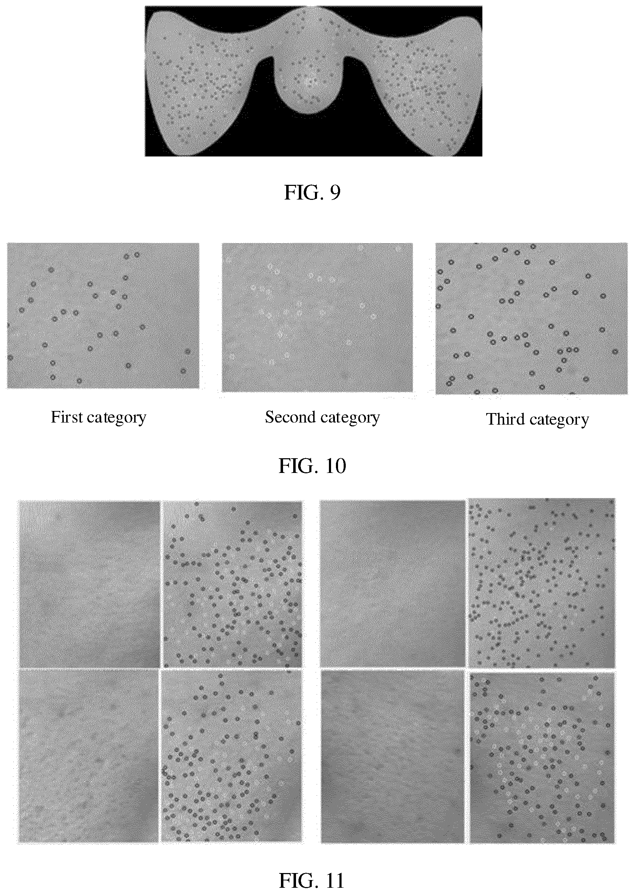

Subsequently, FIG. 9 is a schematic diagram of pores in a target area of a first image according to this application. FIG. 9 shows all pores further determined by the terminal device in the target area after determining the target area of a face in the first image. In this case, the terminal device may extract feature information of each pore shown in FIG. 9.

Optionally, the feature information of the pores in the embodiments of this application includes at least one of the following: an area, a depth, and a color of the pores. In this case, the terminal device may classify the pores into a plurality of different categories based on areas, depths, and colors of all pores in the target area. A manner of determining a pore position and extracting feature information of the pores is not limited in this application. For example, a technology such as image recognition may be used. For example, FIG. 10 is a schematic diagram of pores of a plurality of categories according to this application. An example shown in FIG. 10, a possible pore classification manner is provided. In the classification manner, pores may be classified into three different pore categories: a first category (grade) of pores, a second category (grade) of pores and a third category (grade) of pores. The first category of pores have a larger area, larger color difference between the inside and the outside, and a deeper depth. The second category of pores have a moderate area, moderate color difference between the inside and the outside, and a moderate depth. The third category of pores have a smaller area, smaller color difference between the inside and the outside, and a shallower depth. It should be noted that, in this embodiment, the pores are classified into three categories, and a relative size of each category is used as an evaluation standard of "larger", "smaller", and "moderate", and the evaluation standard may be implemented in a manner such as segmental truncation after size sorting. Therefore, the foregoing manner in which the pores are classified into the three categories is merely an example for description. The pores may be classified into more pore categories, so as to obtain more detailed classification to improve stability and accuracy of pore detection. Implementation principles of the pores are the same, and only a quantity is increased. Details are not described again.

Optionally, in S102, the terminal device may specifically send the extracted feature information of all pores to a pore classification model trained in advance, and all pores are classified by using the pore classification model. The pore classification model may be a classifier implemented based on machine learning, for example, a support vector machine (support vector machine, SVM). Alternatively, the pore classification model may be another classifier implemented based on machine learning. This is not limited in this application. Optionally, in S102, in addition to classifying the pore categories by using the foregoing classifier, the pore categories may be classified in a manner such as threshold classification, ensemble learning (adaboost).

Therefore, the terminal device may input the extracted feature information of all pores in the target area of the face into the pore classification model, to obtain scoring data of the feature information of each pore in the pore classification model, and finally, classify all pores in the target area into the plurality of categories based on the scoring data of the feature information of each pore. The scoring data is an output parameter of the classifier implemented based on machine learning. In this embodiment of this application, the scoring data is a score of similarity between the entered pores and the learned pores of different categories after the pore classification model compares the input feature information of the pores with the learned feature information of the pores. A larger score indicates that the entered pores are more similar to the learned pores.

However, to obtain, by training, the foregoing pore classification model that can be used for classification, the terminal device may perform training in advance based on the marked feature information of the pores of the plurality of pore categories. For example, after obtaining feature information of 1000 pores of the first category, feature information of 1000 pores of the second category, and feature information of 1000 pores of the third category in advance, the terminal device sends the feature information to the machine learning model classifier, to obtain the foregoing pore classification model by training. The pore classification model compares the input feature information of the pores with the trained feature information of the total of 3000 pores, and outputs scoring data, where the scoring data is used to indicate which category of the pores is most similar to the input pores.

For example, FIG. 11 is a schematic diagram of pores that are classified by a pore classification model according to this application. It may be learned that for pores on each image, the pore classification model may classify all pores on each image into three different pore categories: a first category, a second category and a third category in a manner shown in FIG. 10.

S103: Input the feature information of each pore category into at least one pore detection model, to obtain pore detection data of each pore category.

Specifically, the terminal device processes the feature information of the pores that have been classified into the plurality of pore categories in S102, and inputs the feature information of all pores into the at least one pore detection model based on the pore categories to which the pores belong, to obtain the pore detection data corresponding to each pore category.

In a specific implementation of S103, the terminal device may store a plurality of pore detection models that are in a one-to-one correspondence with the pore categories, and the terminal device sends pores of different categories to the pore detection models of the corresponding pore categories based on the categories of all pores in S102, to obtain the detection data corresponding to the pore category.

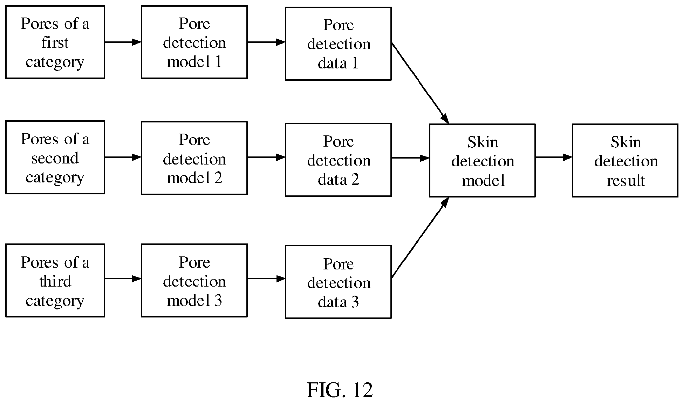

For example, FIG. 12 is a schematic diagram of a structure of a pore detection model according to this application. It is assumed that the terminal device determines, by using S102, that the face in the first image includes 20 pores of the first category, 30 pores of the second category, and 50 pores of the third category. In this case, in S103, the terminal device inputs feature information of the 20 pores of the first category into a pore detection model 1 to obtain output pore detection data 1, inputs feature information of the 30 pores of the second category into a pore detection model 2 to obtain output pore detection data 2, and inputs feature information of the 50 pores of the third category into a pore detection model 3 to obtain output pore detection data 3.

The pore detection model 1, the pore detection model 2, and the pore detection model 3 may alternatively be machine learning classifier models obtained by training in advance, and the terminal device may obtain a plurality of training images including a face. Pores on a face in each training image may be classified into the foregoing three pore categories. Subsequently, the pore detection models that are in a one-to-one correspondence with the pore categories may be trained based on feature information of pores in each of the three pore categories in the plurality of training images, to obtain the pore detection model 1, the pore detection model 2, and the pore detection model 3 that are in one-to-one correspondence with the three pore categories.

A training manner of the pore detection model and a quantity of training images are not limited in this application. For example, 115 established images may be used as training images, and regression models of pores of different categories obtained by training by using a machine learning method are used as corresponding pore detection models. A quantitative score of the pores of the category is obtained by using the pore detection model and is used as the pore detection data.

In another specific implementation of S103, the terminal device may obtain one continuous weight curve through training by using only one regression model, and for each pore, a corresponding weight may be found on the weight curve. A penalty score is set for each pore, so that an output score, for example, between 60 and 100, is obtained in combination with the weight. In this case, the regression model may be recorded as a first pore detection model. For the terminal device, the feature information of the pores of all different categories that is obtained in S102 may be input into the first pore detection model, to obtain detection data of the pores of each pore category, and the detection data may be recorded as the pore detection data 1, the pore detection data 2, and the pore detection data 3.

S104: Determine a skin detection result of the face based on the pore detection data of the plurality of pore categories.

Specifically, in S104, the terminal device jointly determines the skin detection result of the face based on the pore detection data of all the plurality of pore categories that is obtained in S103.

In a specific implementation of S104, referring to FIG. 12, the terminal device jointly inputs the obtained pore detection data 1, the pore detection data 2, and the pore detection data 3 into the skin detection model, and obtains the skin detection result of the face based on output of the skin detection model. The skin detection model may be obtained by training in advance and stored in the terminal device. For example, the skin detection model may set weight coefficients of pores of different pore categories based on different proportions of the pores of the different pore categories in the pore detection model. The weight coefficients may be obtained through training by using the regression model. The training method includes but is not limited to multiple linear regression, support vector regression (SRV), linear regression, and ensemble learning (adaboost).

Optionally, the skin detection result may be skin delicateness of the face. The skin detection model may multiply the pore detection data 1, the pore detection data 2, and the pore detection data 3 that are output by different pore detection models by different weight coefficients, to obtain three products, and add up the three products to obtain a result value. The skin detection result may be classified based on the result value. A result value below 75 corresponds to an average skin test result, a result value from 75 to 85 corresponds to a good skin test result, and a result value above 85 corresponds to an excellent skin test result. It should be noted that division of the result values herein is merely an example for description, rather than a limitation.

Finally, according to the facial skin detection method provided in this embodiment, after the foregoing S101 to S104, the pores in the first image that includes the face and that is obtained by the terminal device by using the front-facing camera may be classified into the plurality of different pore categories. After the feature information of each pore category is detected by using the regression model that is in a one-to-one correspondence with the pore category, the skin detection result of the face is finally determined based on the pore detection data of all pore categories. Therefore, in this embodiment, through the classification detection of the pores in the face image photographed by the front-facing camera, the terminal device can perform skin detection based on the face image photographed by the front-facing camera. In this way, a technical problem in the conventional technology that the terminal device cannot perform skin detection based on the face image photographed by the front-facing camera can be resolved. In addition, this application can enable the terminal device to provide that in different scenarios including the front-facing camera, a user can detect facial skin, and a detection result has certain accuracy and stability. In addition, because the pore detection model and a skin detection model are used, and the method does not depend on a recognition result of a single image, an unreasonable jump does not occur in the detection result when the terminal device performs skin detection on a same face in different scenarios at a same time. In this way, a stable and accurate skin detection result can be provided for the user of the terminal device by using the front-facing camera.

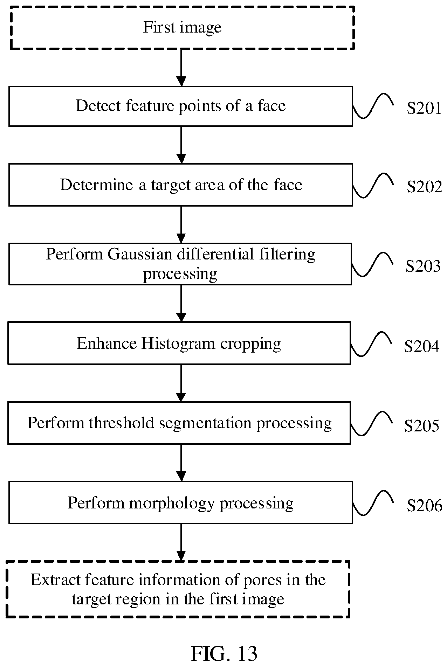

Further, according to the foregoing embodiment, the facial skin detection method is based on the image photographed by the terminal device by using the front-facing camera, and the image photographed by the front-facing camera has relatively low quality, and cannot be directly used to extract the feature information of the pores sometimes. Therefore, this application further provides a method in which a terminal device pre-processes an image after obtaining a first image and before extracting feature information of pores in a target area in the first image, so that the terminal device can more accurately extract the feature information of the pores. Specifically, FIG. 13 is a schematic flowchart of image preprocessing in a facial skin detection method according to this application. As shown in FIG. 13, the method includes the following steps.

S201: Detect a feature point of a face in a first image. In S201, the terminal device first detects the face in the first image, and annotates different feature points of the face in the first image. The feature point may be, for example, an eye, a mouth, or a nose.

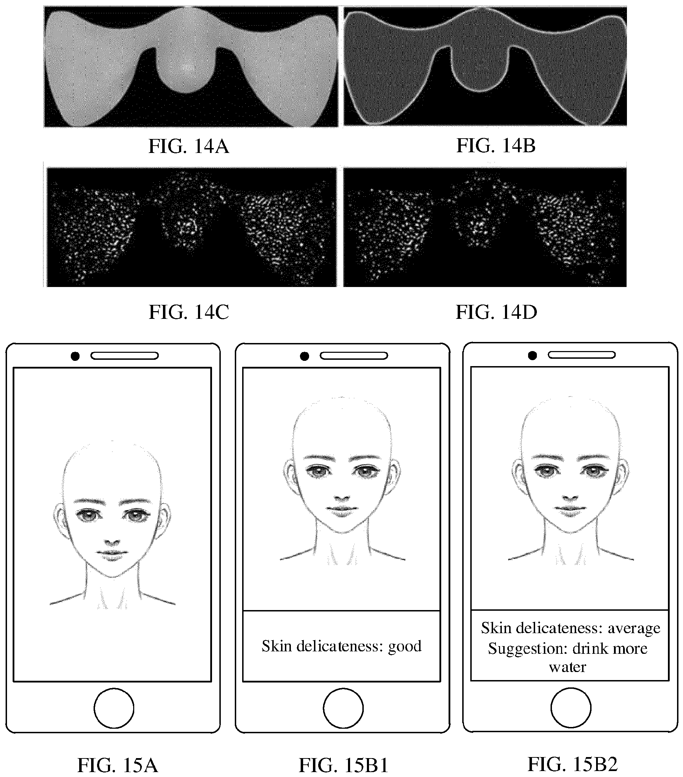

S202: Determine a target area of the face based on the feature point of the face. Subsequently, the terminal device further extracts, based on the feature point that is of the face in the first image and that is detected in S201, the target area used for subsequent skin detection. For example, FIG. 14A, FIG. 14B, FIG. 14C, and FIG. 14D are schematic diagrams of image preprocessing according to this application. FIG. 14A shows a target area extracted by the terminal device.

S203: Perform Gaussian differential filtering processing on the target area. The terminal device performs Gaussian differential filtering processing on the target area in FIG. 14A, so that edge information of dark pores in the target area is clearer. The target area on which Gaussian differential filtering processing is performed is shown in FIG. 14B.

S204: Continue to perform histogram cropping processing on the target area. The terminal device performs histogram cropping on information obtained after Gaussian differential filtering processing, to enhance contrast of pore edges.

S205: Continue to perform threshold segmentation processing on the target area. The terminal device performs, based on the threshold, segmentation on the target area obtained after contrast enhancement, to obtain all pores in the target area shown in FIG. 14C.

S206: Continue to perform morphological processing on the target area. After the terminal device performs morphological processing such as corrosion and expansion on the target area, it is facilitated for the terminal device to further screen some small pores, to obtain all pores in the target area shown in FIG. 14D.

Finally, after processing the first image in S201 to S206 shown in FIG. 13, the terminal device extracts feature information of the pores from the target area of the first image obtained after processing. In this case, for the target area shown in FIG. 14D, the terminal device may extract the feature information of all pores in the target area by using a binarization mask of the pores.

In conclusion, in this embodiment, after preprocessing the first image by using the processing method shown in FIG. 13, the terminal device extracts the feature information of all pores from the target area of the first image obtained after processing. Accuracy of determining, by the terminal device, the pores in the target area and the extracted feature information of the pores can be improved, and therefore, accuracy of subsequent detection on the facial skin can be ensured. In addition, after the preprocessing process shown in FIG. 13 is performed, the terminal device may perform skin detection on the face only by using the target area, and does not need to detect the entire first image. In this way, a calculation amount when the terminal device performs skin detection on the face is reduced, a processing time is reduced, and efficiency of performing facial skin detection by the terminal device can be improved.

Further, in the foregoing embodiment, with reference to a facial skin detection scenario photographed by the front-facing camera to which this application is applied, this application further provides a method for displaying a terminal device by using a display interface. The following describes the method with reference to accompanying drawings.

FIG. 15A, FIG. 15B1, and FIG. 15B2 are schematic diagrams of an embodiment of a method for displaying a display interface of a terminal device according to this application. A display interface shown in FIG. 15A is an interface in which the terminal device performs photographing by using a front-facing camera. When a user starts an application related to skin detection in the terminal device, the terminal device obtains a first image including a user face through photographing by using the front-facing camera, and after a skin detection result of the face is determined by using any one of the foregoing facial skin detection methods in this application, the skin detection result may be displayed below the first image as "Skin delicateness: good" by using the display interface shown in FIG. 15B1. It should be noted that the method for displaying UI interfaces shown in FIG. 15A, FIG. 15B1, and FIG. 15B2 is merely an example for description, and a skin detection result displayed by using another picture, shape, or style also falls within the protection scope of this application.

Optionally, according to the display method provided in this embodiment, in addition to displaying the skin detection result of the face in FIG. 15B2, after determining the skin detection result of the face, the terminal device may further display suggestion information for the user on the display interface, to help the user solve a problem related to the skin detection result. In a possible implementation, after determining the skin detection result of the face, the terminal device may further determine, based on the skin detection result, a problem existing in the skin of the face, and display, on the display interface, at least one piece of suggestion information used to resolve the problem. For example, if the terminal device determines that the skin detection result of the face is average, and further determines that an existing problem is that the face is greasy, the terminal device may simultaneously display, on the display interface shown in FIG. 15B2, a skin detection result as"Skin delicateness: average" and display suggestion information "Suggestion: drink more water".

The terminal device determines the suggestion information in a manner of a prestored mapping relationship. For example, the terminal device may store a correspondence of "average-greasy-drink more water". When determining that the skin detection result of the face is average, it may be determined that the facial skin problem is "greasy" based on the mapping relationship, and finally, the suggestion information "Suggestion: drink more water" is displayed on the display interface. Alternatively, the terminal device may further obtain the suggestion information by interacting with the server for display. For example, after determining the skin detection result of the face, the terminal device sends the skin detection result to the server, where the server may be an electronic device used by medical personnel in a medical cosmetology institution. In this case, after diagnosing the problem existing in the skin of the face based on the skin detection result, the medical personnel may input the suggestion information and send the suggestion information back to the terminal device. In this case, after receiving the at least one piece of suggestion information for resolving the problem that is sent by the server, the terminal device displays, on the display interface shown in FIG. 15B2, the received suggestion information "Suggestion: drink more water".

In conclusion, the facial skin detection method provided in the foregoing embodiments can be used for a display interface of the terminal device. When a user shoots a face image by using the front-facing camera, the terminal device may directly display the skin detection result of the face on the display interface after determining the skin detection result of the face based on the face image. In addition, the terminal device may further obtain the suggestion information based on the skin detection result. Therefore, the terminal device can provide a facial skin detection function and a related consultation function for the user. In this way, more functions can be provided by the terminal device during facial skin detection, and user experience of the terminal device is further improved.

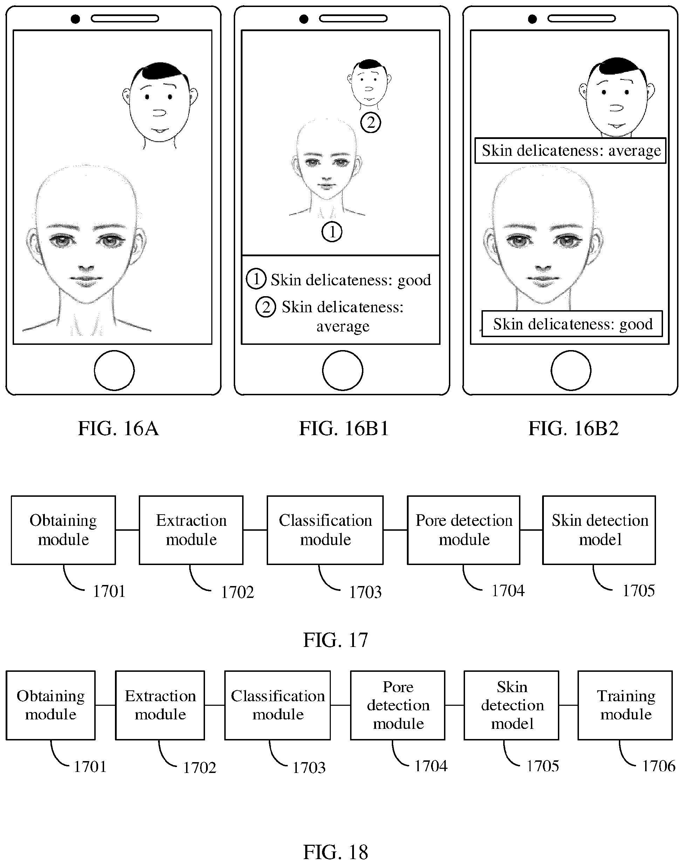

Optionally, according to the foregoing embodiment, this application further provides a multi-person facial skin detection method applicable to a front-facing camera. Corresponding display interfaces of the multi-person facial skin detection method may be shown in FIG. 16A, FIG. 16B1, and FIG. 16B2. FIG. 16A, FIG. 16B1, and FIG. 16B2 are schematic diagrams of another embodiment of a method for displaying a display interface of a terminal device according to this application. This application is applied to a scenario shown in FIG. 16A. If the terminal device detects a plurality of faces by using the front-facing camera when performing facial skin detection, for example, if there are two faces shown in FIG. 16A, after obtaining a first image including the two faces, the terminal device separately determines, by using the methods in S102 and S104 in FIG. 4, skin detection results that are in a one-to-one correspondence with the two faces. For example, a skin detection result of a lower face is "Skin delicateness: good", and a skin detection result of an upper face is "Skin delicateness: average". Subsequently, the terminal device may display the skin detection result "Skin delicateness: good" of a user ① and the skin detection result "Skin delicateness: average" of a user ② at the bottom of the first image on the display interface shown in FIG. 16B1. To facilitate distinguishing between the user ① and the user ②, the terminal device separately displays an identifier ① and an identifier ② of the users at positions corresponding to the two faces in the first image. Alternatively, the terminal device may not need to identify the user, but directly display the first image on the display interface shown in FIG. 16B2, and display, in a one-to-one correspondence with the users on the first image, skin detection results respectively corresponding to the two users.

In conclusion, in the facial skin detection method provided in the foregoing embodiments, after obtaining the image including the plurality of faces, the terminal device may detect the skin of each face in the image, and display the skin detection result of each face on the display interface at the same time after obtaining the skin detection result of each face. In this way, after photographing faces with a family member and a friend by using the front-facing camera of the terminal device, the user can directly view a skin detection result of each person by using the display interface of the terminal device. In this way, more functions can be provided by the terminal device during facial skin detection, interest is added when the user uses the facial skin detection, and user experience of the terminal device can also be improved.

In the foregoing embodiments provided in this application, the facial skin detection method provided in the embodiments of this application is described from a perspective of a terminal device. To implement the functions in the method provided in the embodiments of this application, a terminal device may further include a hardware structure and/or a software module, to implement the functions in a form of a hardware structure, a software module, or a hardware structure and a software module. Whether a specific function in the foregoing functions is performed by the hardware structure, the software module, or the combination of the hardware structure and the software module depends on specific applications and design constraints of the technical solutions.

For example, FIG. 17 is a schematic diagram of a structure of an embodiment of a facial skin detection apparatus according to this application. The apparatus shown in FIG. 17 may be a terminal device, and the apparatus may be configured to perform the facial skin detection method in the foregoing embodiments of this application. The apparatus includes an obtaining module 1701, an extraction module 1702, a classification module 1703, a pore detection module 1704, and a skin detection module 1705. The obtaining module 1701 is configured to obtain a first image including a face. The extraction module 1702 is configured to extract feature information of pores in a target area of the face. The classification module 1703 is configured to classify the pores in the target area into a plurality of pore categories based on the feature information of the pores in the target area. The pore detection module 1704 is configured to input feature information of pores of each pore category into at least one pore detection model to obtain pore detection data of each pore category. The skin detection module 1705 is configured to determine a skin detection result of the face based on pore detection data of the plurality of pore categories.

Optionally, the feature information of the pores includes one or more of the following: an area, a depth, and a color of the pores.

Optionally, the classification module 1703 is specifically configured to input the feature information of all pores into a pore classification model, to obtain scoring data of the feature information of each pore by the pore classification model. The pore classification model is obtained by training the feature information of the pores of the plurality of pore categories. The pores in the target area are classified into a plurality of categories based on the scoring data of the feature information of each pore.

Optionally, the pore detection module 1704 is specifically configured to input the feature information of the pores of each pore category into a plurality of pore detection models that are in a one-to-one correspondence with the pore categories, to obtain detection data of each pore category. The plurality of pore detection models are obtained by training the feature information of the pores of the corresponding pore categories.

Further, FIG. 18 is a schematic diagram of a structure of an embodiment of a facial skin detection apparatus according to this application. Based on the embodiment shown in FIG. 17, the apparatus shown in FIG. 18 further includes a training module 1706. The obtaining module 1701 is further configured to obtain a plurality of images including a face, where pores on a face in each image may be classified into the plurality of pore categories. The training module 1706 is configured to train, based on feature information of pores of each pore category in the plurality of images, a pore detection model that is in a one-to-one correspondence with the pore category, to obtain a plurality of pore detection models that are in a one-to-one correspondence with the plurality of pore categories.

Optionally, the pore detection module 1704 is specifically configured to input the feature information of the pores of each pore category into the first pore detection model, to obtain detection data of each pore category, where the first pore detection model is obtained by training feature information of the pores of all categories.

Optionally, the skin detection module 1705 is specifically configured to input pore detection data of the plurality of pore categories into a skin detection model obtained by training in advance, to obtain a skin detection result of the face, where the skin detection model is established based on a weight coefficient occupied by at least one pore category in the skin detection result.

Optionally, the weight coefficient occupied by the at least one pore category in the skin detection result is obtained through regression model training.

Optionally, the skin detection result includes skin delicateness of the face.

Optionally, the extraction module 1702 is further configured to determine the target area of the face in the first image, sequentially perform filtering processing, enhancement processing and morphology processing on the target area, and determine the feature information of all pores from the processed target area.

Optionally, the obtaining module 1701 is specifically configured to obtain, by using the front-facing camera, the first image including the face.

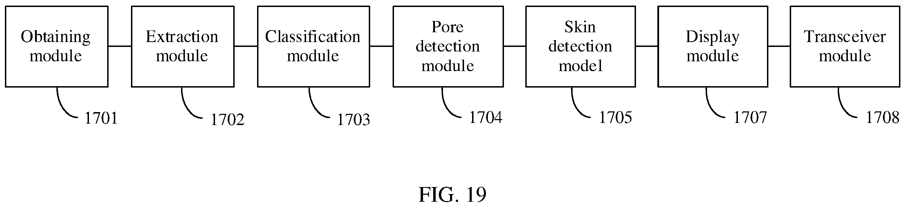

Further, FIG. 19 is a schematic diagram of a structure of an embodiment of a facial skin detection apparatus according to this application. Based on the embodiment shown in FIG. 17 or FIG. 18, the apparatus shown in FIG. 19 further includes a display module 1707 and a transceiver module 1708.

Optionally, the skin detection module 1705 is further configured to determine a problem existing in the facial skin and at least one piece of suggestion information for resolving the problem. The display module 1707 is further configured to display, on a display interface, the at least one piece of suggestion information for resolving the problem.

Optionally, the transceiver module 1708 is configured to send the skin detection result to a server, where the server is configured for medical personnel to diagnose the problem existing in the facial skin. The transceiver module 1708 is further configured to receive at least one piece of suggestion information for resolving the problem that is sent by the server. The display module 1707 is configured to display, on a display interface, the at least one piece of suggestion information for resolving the problem.

Optionally, the first image includes a plurality of faces. The skin detection result of the face includes: a skin detection result that is in a one-to-one correspondence with each face in the first image.

Optionally, the display module 1707 is further configured to: display the first image on a display interface, and display, at a position corresponding to each face in the first image, a skin detection result that is in a one-to-one correspondence with each face.

Division into modules in embodiments of this application is an example, and is merely logical function division. During actual implementation, there may be another division manner. In addition, functional modules in embodiments of this application may be integrated into one processor, or each of the modules may exist alone physically, or two or more modules are integrated into one module. The integrated module may be implemented in a form of hardware, or may be implemented in a form of a software functional module.

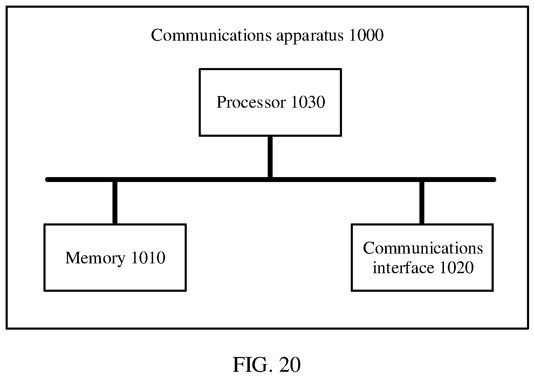

FIG. 20 is a schematic diagram of a structure of an embodiment of a facial skin detection apparatus according to this application. The apparatus may be a terminal device. The apparatus 1000 shown in FIG. 20 includes a memory 1010, configured to store a program. The communications interface 1020 is configured to communicate with another device. The processor 1030 is configured to execute the program in the memory 1010. It should be understood that the video data processing apparatus shown in FIG. 20 may perform steps performed by the terminal device in the embodiments.

For example, when the facial skin detection apparatus 1000 is configured to perform the steps of the facial skin detection method performed by the terminal device in the foregoing embodiments of this application, and when the program stored in the memory 1010 is executed, the processor 1030 may obtain, by using the communications interface 1020, a first image including a face. The processor 1030 is further configured to: extract feature information of pores in the target area of the face, classify the pores in the target area into a plurality of pore categories based on the feature information of the pores in the target area, and input the feature information of the pores in each pore category into at least one pore detection model, to obtain pore detection data of each pore category, so as to determine a skin detection result of the face based on the pore detection data of the plurality of pore categories.

For another example, the processor 1030 may further obtain, by using the communications interface 1020, a plurality of images including a face, where pores on a face in each image may be classified into the plurality of pore categories. The processor 1030 is further configured to train, based on feature information of pores of each pore category in the plurality of images, a pore detection model that is in a one-to-one correspondence with the pore category, to obtain a plurality of pore detection models that are in a one-to-one correspondence with the plurality of pore categories.

For another example, the processor 1030 may be further configured to determine the target area of the face in the first image, sequentially perform filtering processing, enhancement processing, and morphology processing on the target area, and determine the feature information of all pores from the processed target area.

For another example, the apparatus shown in FIG. 20 may further include: a display apparatus, configured to display, on a display interface, at least one piece of suggestion information for resolving the problem.

It should be understood that the apparatus 1000 shown in FIG. 20 may be a chip or a circuit. For example, a chip or a circuit that may be disposed in a terminal device. The communications interface 1020 may alternatively be a transceiver. The transceiver includes a receiver and a transmitter. Further, the communications apparatus 1000 may further include a bus system.

The processor 1030, the memory 1010, the receiver, and the transmitter are connected to each other through the bus system. The processor 1030 is configured to execute an instruction stored in the memory 1010, to control the receiver to receive a signal and control the transmitter to send a signal, and complete the steps performed by the network device in the communications method in this application. The receiver and the transmitter may be a same physical entity or different physical entities. When being the same physical entity, the receiver 240 and the transmitter 250 may be collectively referred to as a transceiver. The memory 1010 may be integrated in the processor 1030, or may be disposed separately from the processor 1030.

In an implementation, it may be considered that functions of the receiver and the transmitter are implemented by using a transceiver circuit or a dedicated transceiver chip. The processor 1030 may be implemented by a dedicated processing chip, a processing circuit, a processor, or a general-purpose chip.

Persons of ordinary skill in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments, and details are not described herein again.

In the embodiments provided in this application, it should be understood that the described apparatus embodiments are merely examples. For example, division into the units is merely logical function division and may be other division in actual implementation. For example, a plurality of units or components may be combined or integrated into another system, or some features may be ignored or not performed. In addition, the displayed or discussed mutual communication connections may be implemented through some interfaces. The indirect couplings or communication connections between the apparatuses or units may be implemented in electronic, mechanical, or other forms.

In addition, units in the apparatus embodiments of this application may be integrated into one processing unit, or each of the units may exist alone physically, or two or more units may be integrated into one unit.

It may be understood that, the processor in the embodiments of this application may be a central processing unit (central processing unit, CPU), or may be another general-purpose processor, a digital signal processor (digital signal processor, DSP), an application-specific integrated circuit (application specific integrated circuit, ASIC), a field programmable gate array (field programmable gate array, FPGA) or another programmable logic device, a transistor logic device, a hardware component, or any combination thereof. The general-purpose processor may be a microprocessor or any conventional processor.

All or some of the methods in the embodiments of this application may be implemented by software, hardware, firmware, or any combination thereof. When software is used to implement embodiments, all or some of embodiments may be implemented in a form of a computer program product. The computer program product includes one or more computer programs or instructions. When the computer programs or instructions are loaded and executed on a computer, all or some of the procedures or functions in the embodiments of this application are performed. The computer may be a general purpose computer, a dedicated computer, a computer network, or another programmable apparatus. The computer programs or instructions may be stored in a computer-readable storage medium, or may be transmitted via the computer-readable storage medium. The computer-readable storage medium may be any usable medium accessible by a computer, or a data storage device, such as a server integrating one or more usable media. The usable medium may be a magnetic medium, for example, a floppy disk, a hard disk, or a magnetic tape, or may be an optical medium, for example, a CD-ROM or a DVD, or may be a semiconductor medium, for example, a solid state disk (solid state disk, SSD), a random access memory (random access memory, RAM), a read-only memory (read-only memory, ROM), and a register.

For example, a storage medium is coupled to a processor, so that the processor can read information from the storage medium and write information into the storage medium. Certainly, the storage medium may alternatively be a component of the processor. The processor and the storage medium may be located in an ASIC. In addition, the ASIC may be located in a network device or a terminal device. Certainly, the processor and the storage medium may alternatively exist in a sending device or a receiving device as discrete components.

In the embodiments of this application, unless otherwise stated or there is a logic conflict, terms and/or descriptions between different embodiments are consistent and may be mutually referenced, and technical features in the different embodiments may be combined based on an internal logical relationship thereof, to form a new embodiment.

It may be understood that, various numerals in the embodiments of this application are merely used for distinguishing for ease of description, and are not intended to limit the scope of the embodiments of this application. The sequence numbers of the foregoing processes do not mean an execution sequence, and the execution sequence of the processes should be determined according to functions and internal logic of the processes.

## Claims

1. A facial skin detection method, comprising:
obtaining a first image comprising a face;
extracting feature information of pores in a target area of the face, and classifying the pores in the target area into a plurality of pore categories based on the feature information of the pores in the target area;
inputting feature information of pores of each pore category into at least one pore detection model, to obtain pore detection data of each pore category; and
determining a skin detection result of the face based on pore detection data of the plurality of pore categories.

2. The method according to claim 1, wherein the feature information of the pores comprises one or more of the following: an area, a depth, and a color of the pores.

3. The method according to claim 2, wherein the classifying the pores in the target area into a plurality of pore categories based on the feature information of the pores in the target area comprises:
inputting the feature information of all pores into a pore classification model, to obtain scoring data of feature information of each pore by the pore classification model, wherein the pore classification model is obtained by training the feature information of the pores of the plurality of pore categories; and
classifying the pores in the target area into the plurality of categories based on the scoring data of the feature information of each pore.

4. The method according to claim 3, wherein the inputting feature information of pores of each pore category into at least one pore detection model, to obtain detection data of each pore category comprises:
inputting the feature information of the pores of each pore category into a plurality of pore detection models that are in a one-to-one correspondence with the pore categories, to obtain the detection data of each pore category, wherein the plurality of pore detection models are obtained by training the feature information of the pores of the corresponding pore categories.

5. The method according to claim 4, further comprising:
obtaining a plurality of images comprising a face, wherein pores on a face in each image may be classified into the plurality of pore categories; and
training, based on feature information of pores of each pore category in the plurality of images, a pore detection model that is in a one-to-one correspondence with the pore category, to obtain a plurality of pore detection models that are in a one-to-one correspondence with the plurality of pore categories.

6. The method according to claim 3, wherein the inputting feature information of pores of each pore category into at least one pore detection model, to obtain detection data of each pore category comprises:
inputting the feature information of the pores of each pore category into a first pore detection model, to obtain the detection data of each pore category, wherein the first pore detection model is obtained by training the feature information of the pores of all categories.

7. The method according to any one of claims 1 to 6, wherein the determining a skin detection result of the face based on pore detection data of the plurality of pore categories comprises:
inputting the pore detection data of the plurality of pore categories into a skin detection model obtained by training in advance, to obtain the skin detection result of the face, wherein the skin detection model is established based on a weight coefficient occupied by the at least one pore category in the skin detection result.

8. The method according to claim 7, wherein the weight coefficient occupied by the at least one pore category in the skin detection result is obtained through regression model training.

9. The method according to any one of claims 1 to 8, wherein
the skin detection result comprises skin delicateness of the face.

10. The method according to any one of claims 1 to 9, wherein before the extracting feature information of pores in a target area of the face, the method further comprises:
determining the target area of the face in the first image;
sequentially performing filtering processing, enhancement processing, and morphology processing on the target area; and
determining the feature information of all pores from the processed target area.

11. The method according to any one of claims 1 to 10, wherein the obtaining a first image comprising a face comprises:
obtaining, through photographing by using a front-facing camera, the first image comprising the face.

12. The method according to any one of claims 1 to 11, wherein after the determining a skin detection result of the face, the method further comprises:
determining a problem existing in facial skin and at least one piece of suggestion information for resolving the problem; and
displaying, on a display interface, the at least one piece of suggestion information for resolving the problem.

13. The method according to any one of claims 1 to 11, wherein after the determining a skin detection result of the face, the method further comprises:
sending the skin detection result to a server, wherein the server is configured for medical personnel to diagnose a problem existing in facial skin;
receiving at least one piece of suggestion information for resolving the problem that is sent by the server; and
displaying, on a display interface, the at least one piece of suggestion information for resolving the problem.

14. The method according to any one of claims 1 to 13, wherein
the first image comprises a plurality of faces; and
the skin detection result of the face comprises: a skin detection result that is in a one-to-one correspondence with each face in the first image.

15. The method according to claim 14, further comprising:
displaying the first image on the display interface, and displaying, at a position corresponding to each face in the first image, the skin detection result that is in a one-to-one correspondence with each face.

16. A facial skin evaluation apparatus, configured to implement the method according to any one of claims 1 to 15.

17. An apparatus, comprising a processor and a memory, wherein the memory stores instructions, and when the processor invokes the instructions, the apparatus is enabled to perform the method according to any one of claims 1 to 15.

18. A computer-readable storage medium, comprising instructions, wherein when the instructions are run on a computer, the computer is enabled to perform the method according to any one of claims 1 to 15.

19. A program product, wherein the program product comprises a computer program, the computer program is stored in a readable storage medium, at least one processor of a communications apparatus can read the computer program from the readable storage medium, and the at least one processor executes the computer program to enable the communications apparatus to implement the method according to any one of claims 1 to 15.
